# EUROPEAN PATENT APPLICATION

(11) **EP 4 744 609 A1**
(43) Date of publication of application: **20.05.2026**
(21) Application number: 25216561.8
(22) Date of filing: 18.11.2025
(51) Int. Cl.: A61B 34/10, A61B 34/00, A61B 18/14

(54) **CLASSIFICATION OF ABLATION POINTS ACCORDING TO CATHETER DATA**

(30) Priority: 19.11.2024 US 202463722319 P; 24.10.2025 US 202519368207
(71) Applicant: Biosense Webster (Israel) Ltd., 2066717 Yokneam (IL)
(72) Inventor: AMIT, Matityahu, 2066717 Yokneam (IL); MEIRI, Itay, 2066717 Yokneam (IL); PFEFFER, Shmuel Yitzhak, 2066717 Yokneam (IL)
(74) Representative: Carpmaels & Ransford LLP

(57) **Abstract**

A method includes receiving an anatomical map of wall tissue of at least a portion of a cardiac chamber, the map comprising different anatomical regions. A plurality of ablation points generated by a multi-electrode catheter during an ablation instance can be received. Using a classification algorithm that considers catheter shape data, an anatomical region is identified and all of the ablation points can be classified as belonging to the identified anatomical region. Respective ablation tags can also be presented on the anatomical map according to the classified ablation points and catheter shape data.

## Description

### FIELD OF THE DISCLOSURE

This disclosure relates generally to cardiac ablation and specifically to systems and methods for generating ablation maps.

### BACKGROUND OF THE DISCLOSURE

Ablation maps, which are typically 3D renderings of at least a portion of an organ that can be superimposed with ablation tags, are used by physicians in performing ablation treatments and verifying their efficacy. The 3D rendering may be an electro-anatomical map or an anatomical surface, such as of an inner wall of a cardiac chamber. Ablation indications on the map may assist a physician in assessing the status of an ablation treatment or in identifying properties associated with an ablation site. For example, a physician can use ablation indications to determine whether a sufficient number of ablations were performed in a desired area, or to locate a gap in an ablated tissue path that needs to be closed with further ablations to eliminate an arrhythmia. To enable simple identification of ablation properties, and for generating the required information for a treatment report and/or for research purposes, an appropriate indication of the region where the treatment was applied is beneficial.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, in accordance with an example of the present disclosure;
Fig. 2 is a schematic pictorial illustration of the classification of an ablation point according to catheter data, in accordance with an example of the present disclosure;
Fig. 3 is an ablation map of a cardiac chamber derived using one of the disclosed algorithms for classification of ablation points, in accordance with an example of the present disclosure; and
Fig. 4 is a flow chart that schematically illustrates a method for generating an ablation map of a cardiac chamber by classifying ablation points according to catheter data, in accordance with an example of the present disclosure.

### DETAILED DESCRIPTION OF EXAMPLES

### OVERVIEW

In a cardiac ablation procedure, such as pulmonary vein isolation (PVI) to treat atrial fibrillation, a physician can ablate tissue in a specific anatomical region (e.g., over an entire circumference of an ostium of a PV). The ablation, such as one using a Pulse Field Ablation (PFA) or radiofrequency (RF) technique, may require several iterations at that region to cover the entire circumference of the ostium fully. Each iteration can require moving a multi-electrode ablation catheter to areas of that region deemed by the physician as insufficiently ablated or not ablated at all.

The physician may be aided with an ablation map comprising an anatomical 3D map of the region that also displays the classification of one or more ablation tags to indicate where ablation has already been performed.

However, this classification can be challenging for some anatomies. For example, in the left atrium (LA), the classification can be difficult when:
1. Locating the catheter in the adjacent superior or inferior vein generates closely-spaced, adjacent ablation points, making identification of each location challenging to classify, particularly when the catheter positioning may cause LA wall movement (i.e., tenting effect);
2. Identifying whether the goal of an ablation treatment is isolating a pulmonary vein (PV) or generating a separation line of ablation (e.g., on a posterior LA wall or on a roof wall of the LA) near the PV.

The above example challenges are intensified with PFA multi-electrode ablation since each ablation sequence is performed for a specific anatomical region, but some of the electrodes may be located outside of that region. Thus, connecting a set of resulting ablation points (e.g., from several iterations of ablation) would not make much sense since each set of ablation points made by a multi-electrode catheter at a given time needs to be treated as a whole set and not point-by-point, in which the points may be mixed with other ablation points.

The issues mentioned above, along with others, can cause the ablation tags to fail in accurately indicating the correct location of ablation. This can also lead to falsely showing certain locations as ablated when they were not actually ablated.

For example, any one or more of the above reasons may cause an ablation tag created during the ablation of a superior PV ostium tissue location to incorrectly mark an inferior PV ostium location. Such errors on an ablation map may mislead or make it hard for a physician to decide whether further ablation is necessary.

Examples of the present disclosure described herein provide ablation point classification techniques that can eliminate tagging inaccuracies caused by the abovementioned issues. A set of ablation points, as described in this disclosure, can be one of (i) a set of the catheter electrodes' locations during an instance of ablation with any additional information assigned to the electrodes therein, such as a contact force, an ablation power, and an ablation duration; and (ii) a set of planned ablation points to be generated by the multi-electrode catheter during an instance of ablation.

The algorithms described in more detail below can classify a set of ablation points from an instance of ablation performed by a multi-electrode catheter, considering the specific location of the ablated region and a shape of the catheter (e.g., a circular catheter). The disclosed method can consider ablation points generated at any given instance as a group of points, and not single unrelated points, which can assist in constraining with high confidence the tissue locations of the ablation points to a correct anatomical region or location (e.g., a correct ostium) on the anatomical map.

Additional data related to the catheter, such as a contact force or a tissue proximity index (TPI) of an electrode to cardiac wall tissue, may also be used in the classification.

In a first example of the disclosed technique, a processor can run the following steps of a majority voting algorithm:
1. Assign the ablation instance to the anatomical region (e.g., a given ostium) where most of the catheter's electrodes are deemed to be in contact during that ablation; and
2. Using the catheter location and the shape of the catheter in space, identify the tissue locations of the electrodes in contact with the tissue at that region to generate correct ablation tags.

In a second example, the processor can run the following centroid mapping algorithm steps:
1. Calculate the geometric center (centroid) of all electrodes of the catheter that are in contact with tissue during the ablation sequence; and
2. Assign the ablation points to the region where this centroid is located, considering the catheter shape in space, e.g., constraining the tissue locations of the electrodes in contact according to the catheter model of electrode positions, such as on a circular curve of a loop catheter around the centroid point located inside a given ostium.

In a third example, the processor can run the following weighted voting algorithm steps:
1. Assign weights to each of the catheter's electrodes based on factors like contact quality and/or signal strength; and
2. Using the catheter location and the catheter's shape in space, identify the tissue locations corresponding to the catheter's electrodes with the highest total weight (e.g., a combined sum of assigned weights for a particular electrode or electrode group).

In a fourth example, the processor can run the following probabilistic assignment algorithm steps:
1. Assign each ablation point a probability of belonging to each of a plurality of regions based on a distance from the respective region;
2. Aggregate the probabilities to determine the most likely region associated with the ablation points; and
3. Using the catheter's location and shape in space, constrain the tissue locations of the ablation points to the most likely region.

In a fifth example, the processor can run a machine learning (ML) classification algorithm that can be trained on existing and labelled ablation data to predict the anatomical region for each ablation sequence (e.g., group of ablation points) based on electrode configurations and other previously mentioned data (e.g., contact force and/or catheter shape).

In a sixth example, the processor can use the output from any one or more of the first to fifth algorithms to train another ML-based algorithm, such as a random forest, to achieve the best overall result.

### SYSTEM DESCRIPTION

The following detailed description should be read with reference to the drawings, in which like elements in different drawings are identically numbered. The drawings, which are not necessarily to scale, depict selected examples and are not intended to limit the scope of the disclosure. The detailed description illustrates by way of example, not by way of limitation, the principles of the disclosure. This description will clearly enable one skilled in the art to make and use the invention, and describes several examples, adaptations, variations, alternatives and uses of the invention, including what is presently believed to be the best mode of carrying out the invention.

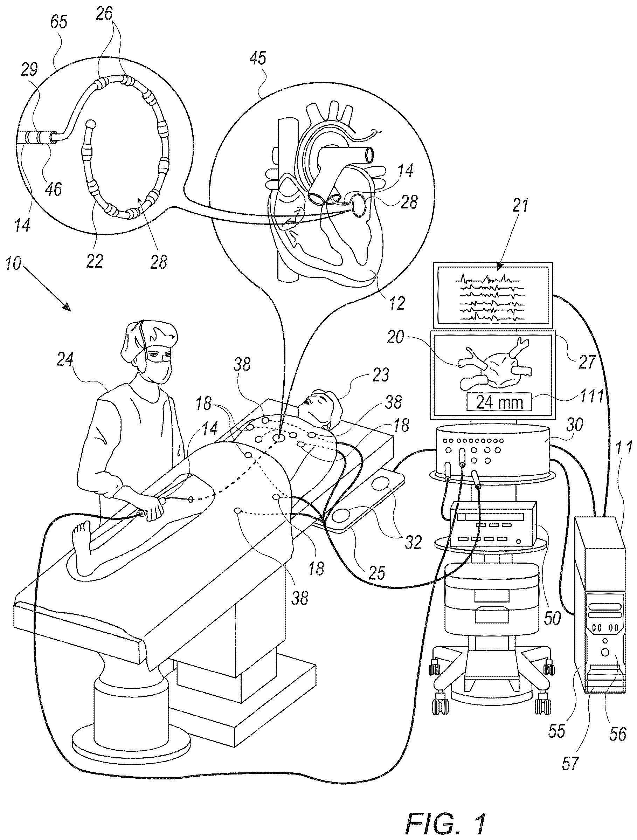

Fig. 1 is a schematic, pictorial illustration of a catheter-based anatomical/electroanatomical (EA) mapping and ablation system 10, in accordance with an example of the present disclosure.

System 10 can include multiple catheters, which can be percutaneously inserted by a physician 24 through the patient's vascular system into a chamber or vascular structure of a heart 12 (seen in inset 45). In some examples, a delivery sheath catheter can be inserted into a cardiac chamber, such as the left or right atrium near a desired location in heart 12. Afterward, a plurality of catheters can be inserted into the delivery sheath catheter to arrive at the desired location. The plurality of catheters may include a catheter dedicated to pacing, a catheter for sensing intracardiac electrogram signals, a catheter dedicated to ablating and/or a catheter dedicated to both EA mapping and ablating. An example loop ablation catheter 14 is illustrated in Fig. 1. In one aspect, loop catheter 14can be configured for sensing bipolar electrograms and applying PFA. Physician 24 can bring a distal end assembly 28 of catheter 14 into contact with a heart wall to ablate a target site in heart 12.

As depicted in inset 65, a distal end assembly 28 of catheter 14 can include multiple electrodes 26 distributed over a curved spline 22, in some examples. Catheter 14 may include a position sensor 29, embedded in or near distal end assembly 28 on a shaft 46 of catheter 14, to track the position and orientation of distal end assembly 28. Optionally, and preferably, position sensor 29 can be a magnetic-based position sensor with three magnetic coils for sensing three-dimensional (3D) position and orientation.

The magnetic-based position sensor 29 may be operated with a location pad 25 that can include a plurality of magnetic coils 32 configured to generate magnetic fields in a predefined working volume. The real-time position of distal end assembly 28 of catheter 14 may be tracked based on magnetic fields generated with location pad 25 and sensed by magnetic-based position sensor 29. Details of the magnetic-based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091.

In another aspect, system 10 can include one or more electrode patches 38 positioned for skin contact on patient 23 to establish a location reference for location pad 25 as well as impedance-based tracking of electrodes 26. For impedance-based tracking, electrical current can be directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 can display cardiac signals 21 (e.g., electrograms acquired at respectively tracked cardiac tissue positions) acquired with body surface ECG electrodes 18 and intracardiac electrograms acquired with electrodes 26 of catheter 14, in some examples. Recorder 11 may include pacing capability to pace the heart rhythm and/or may be electrically connected to a standalone pacer.

System 10 can also include an ablation energy generator 50 adapted to conduct ablative energy to one or more electrodes 26 configured for ablation. The energy produced by ablation energy generator 50 may include but is not limited to, radio frequency (RF) energy or pulse field (PF) energy, including monopolar or bipolar high-voltage DC pulses (i.e., PFA).

The patient interface unit (PIU) 30 is an interface configured to establish electrical communication between catheters, electrophysiological equipment, a power supply, and a workstation 55 to control system 10 operation and receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 can additionally include processing capability for implementing real-time computations of catheter locations and for performing ECG calculations.

Workstation 55 can include a memory 57, a processor unit 56 with memory or storage with appropriate operating software loaded therein, and user interface capability. In one aspect, workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27 such as places where ablation energy has been applied. One commercial product embodying elements of system 10 is available as the CARTOTM 3 System, available from Biosense Webster, Inc.

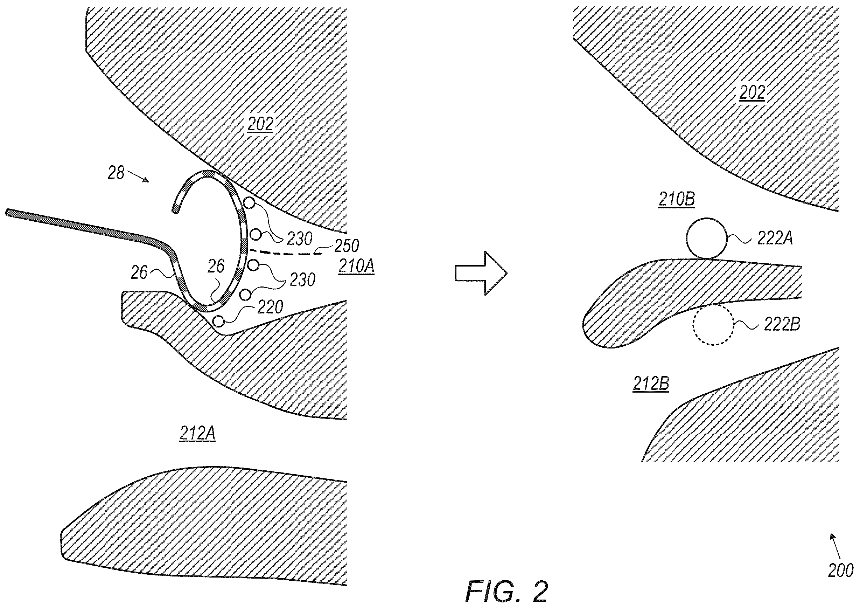

In the disclosed example, processor 56 can run an algorithm that classifies multiple ablation points, like points associated with each electrode of the entire loop distal end assembly 28 of catheter 14, as a group together - considering the specific location of the distal end assembly 28 and its spatial configuration (i.e., shape), which constrains the tissue locations to conform with the catheter loop shape, as shown in Fig. 2.

In some examples, processor 56 can comprise a general-purpose computer programmed in software to perform the functions described herein. The software may be downloaded to the computer in electronic form over a network, for example, or it may, alternatively or additionally, be provided and/or stored on non-transitory tangible media, such as magnetic, optical, or electronic memory.

This configuration of system 10 is shown by way of example to illustrate certain problems addressed by examples of the present disclosure and to demonstrate the application of these examples in enhancing the performance of such a system. However, examples of the present disclosure are not limited to this specific example system, and the principles described herein may similarly be applied to other medical systems. For example, the systems, methods, and techniques described here can be used in conjunction with other multi-electrode ablation catheter types, such as a basket catheter, a balloon catheter, or a multi-electrode ablation catheter of some other suitable shape.

### CLASSIFICATION OF ABLATION POINTS ACCORDING TO CATHETER DATA

Fig. 2 is a schematic pictorial illustration of the classification of an ablation point 220 based at least in part on data received from catheter 14, in accordance with an example of the present disclosure.

As Fig. 2 shows, the flexible tissue of anatomical regions like ostiums 210A and 212A can respond to contact with the catheter (e.g., tenting or deformations), which may cause the tissue locations of ablation to be wrongly reflected (e.g., tagged) in the mapped (200) representation 210B and 212B of the ostiums.

In the shown example, the ablation point 220 can occur at a pushed wall of tissue 202 of ostium 210A. Based on map 200 coordinates only, an algorithm could wrongly assign ablation point 220 with a tag 222B, which islocated at an inferior ostium 212B on the map 200.

By applying one or more of the algorithms discussed above that can take into account the shape of distal end assembly 28, the disclosed techniques can correctly assign ablation point 220 with an ablation tag 222A located at mapped ostium 210B instead of wrongly assigning the ablation tag 222B at ostium 212B.

In particular, in the shown example, the disclosed techniques can accurately assign a correct ablation tag 222A by considering the location of other ablation points 230 performed by the loop catheter during the same ablation instance. In one example, using one of the above-mentioned classification algorithms can comprise determining a correct anatomical region for association with the ablation point by deeming that a centroid location 250 of the ablation points (220, 230) falls in that region.

The disclosed techniques can similarly ensure the ablation tags of other ablation lines, such as one made on the posterior wall of the left atrium, are correctly placed. In this example, the tags will be correctly located despite the flexible posterior wall tissue coordinates changing between those recorded during ablation and ones represented by the map (i.e., representing the tissue in the absence of the catheter and while experiencing no deflections or tenting).

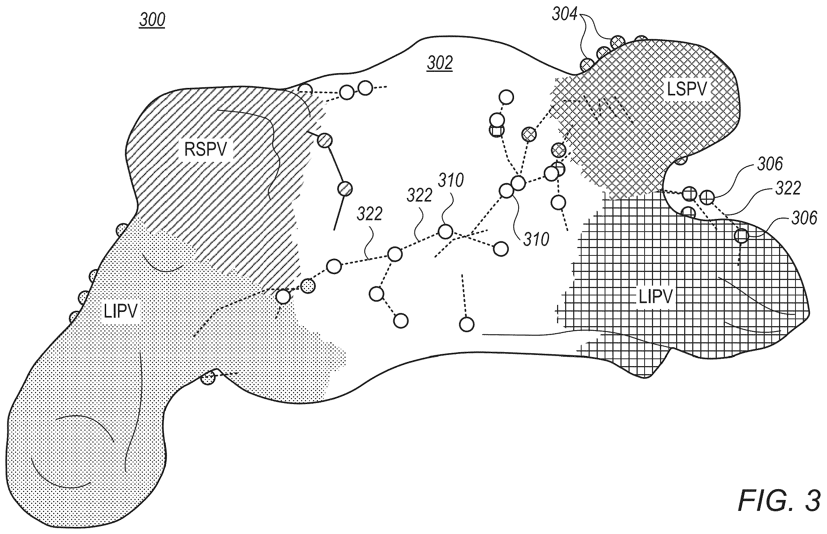

An ablation session can include multiple instances of the sort shown in Fig. 2. An ablation map based on the above analysis applied to multiple ablation instances is depicted in Fig. 3.

### ABLATION MAP BASED ON ABLATION POINTS CLASSIFIED ACCORDING TO CATHETER DATA

Fig. 3 is an ablation map 300 of a cardiac chamber derived using one of the disclosed algorithms for classification of ablation points, in accordance with an example of the present disclosure. Ablation map 300 can include an anatomical map 302 presented (e.g., superimposed) with ablation tags, such as tags 304, 306 and 310.

The depicted chamber is a left atrium, which can be graphically encoded by a segmentation algorithm to visualize, among other things:
the left atrium wall, and the four PVs;
the left superior PV (LSPV);
the left inferior PV (LIPV);
the right superior PV (RSPV); and/or
the right inferior PV (RIPV)

Map 300 can also be based on one or more of the disclosed classification algorithms assigning each set of ablation points of a loop catheter 14 to a correct region, based on catheter data. As seen, the respective ablation tags 304, 306 and 310 can appear as sets, each set distributed over the circular contours (322) of distal end assembly 28. It should be noted that as the tissue returns to its normal shape (i.e., the catheter is repositioned after applying ablative energy such that it is no longer causing the tissue to deform or tent), arcs of the contours may appear as straight lines rather than loops, as in the case between ablation tags 310 of an ablation line over the left atrium wall.

Map 300 can be generated offline using stored data or generated during a clinical ablation procedure and updated in real-time as the ablation procedure advances. Depending on the shape of the catheter, map 300 may have a slightly different appearance than the one exemplified.

### METHOD FOR GENERATING AN ABLATION MAP USING CLASSIFICATION OF ABLATION POINTS

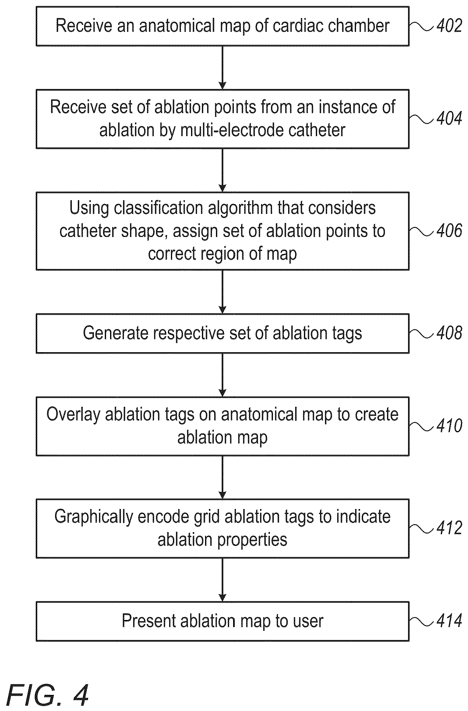

Fig. 4 is a flow chart that schematically illustrates a method for generating an ablation map of a cardiac chamber using the techniques described above for classification of ablation points, in accordance with an example of the present disclosure. One or more of the described algorithms, according to the presented example, can carry out a process that begins with processor 56 receiving anatomical map 302, at a map receiving step 402.

The processor can further receive a set of ablation points from an instance of ablation by a catheter 14 inside the cardiac chamber, at a receiving ablation points step 404. Step 404 can optionally include the application of ablative energy to tissue by catheter 14 as a precursor to receiving the set of ablation points.

Next, the processor can apply one or more of the disclosed classification algorithms that consider the catheter's shape before assigning the ablation points to a correct region of the map, at a classification step 406.

At ablation tags generation step 408, the processor can generate a respective set of ablation tags. The processor can overlay the set of ablation tags 306 on map 302 to generate the ablation map 300, at an ablation tags overlaying step 410.

The processor can graphically encode ablation tags 306 to indicate an ablation property such as PV ostium identity, and level of ablation, at graphical encoding step 412. Graphically encoding the tag ablation map may comprise coloring ablation tags over one region with one color and coloring ablation tags over another region with another color.

Finally, the processor can present the graphically encoded ablation map 300 to a user on the display device 27, at ablation map presentation step 414.

The flow chart in Fig. 4 is simplified to describe a single instance of ablation. However, in an actual procedure, multiple ablation instances may occur (for example, iteratively performing multiple ablations at step 404), leading the processor to update the ablation map 300 numerous times during the procedure. A final map 300 may reflect a complete treatment if the ablation session was fully executed.

Map 300 may be generated using offline data, or generated during a clinical ablation procedure and updated in real time as the ablation procedure advances.

### EXAMPLES

In one example, a method in accordance with the present description can include receiving an anatomical map (200) of wall tissue (202) of at least a portion of a cardiac chamber, the map comprising different anatomical regions (210, 212). The method can further include receiving one or more of a set of ablation points (220, 230) generated by a multi-electrode catheter (14) and a set of planned ablation points to be generated by the multi-electrode catheter (14) at an ablation instance at one of the anatomical regions. Using a classification algorithm that considers catheter (14) shape and location data, the one (210) of the anatomical regions (210, 212) can be identified and all of the ablation points (220, 230) can be classified as belonging to the identified anatomical region (210). Respective ablation tags (222A) can be presented on the anatomical map (200) according to the classified ablation points (220, 230) and catheter shape and location data.

In further embodiments, the classification algorithm can further include determining the correct anatomical region with which to associate the ablation points by deeming that a majority of the ablation points falls (220, 230) in that region.

In other embodiments, the classification algorithm can include determining the correct anatomical region (220) with which to associate the ablation points by deeming that a centroid (250) location of the ablation points and/or the electrodes distributed about the shape of the catheter's distal end falls in that region.

Alternatively or additionally, the classification algorithm can include assigning a weight to each ablation point associated with the catheter (14) shape data and identifying the correct anatomical region (210) with which to associate the ablation points by determining in what region a subset of ablation points (220, 230) yielding the highest total weight are located.

In other embodiments, the classification algorithm can comprise determining the proper anatomical region (210) with which to associate the ablation points by calculating the distance between each of a plurality of ablations points (220, 230) from each of a plurality of regions, and determining the correct region (210) on the map (200) with which to associate the ablation points based on a probability derived from the distances.

In still other embodiments, the classification algorithm can comprise training a machine learning (ML) based algorithm based on a data set of labeled ablation points and using the trained ML-based algorithm to infer the correct anatomical region (210) based on the received set of ablation points (220, 230).

Training the ML model can comprise training the model with data labeled using one or more given classification algorithms among the list of: majority voting classification algorithm, centroid location classification algorithm, distance-based probabilistic classification algorithm, and weighted ablation-points-based classification algorithm (each of which is described herein).

In another example, a system (10) in accordance with the present description can include a memory (57) and a processor (56). The memory can be configured to store a classification algorithm. The processor can be further configured to (i) receive an anatomical map (200) of wall tissue (202) of at least a portion of a cardiac chamber, the map comprising different anatomical regions (210, 212), (ii) receive one or more of a set of ablation points (220, 230) generated by a multi-electrode catheter (14) and a set of planned ablation points to be generated by the multi-electrode catheter (14) at an ablation instance at one of the anatomical regions. In one aspect, using the classification algorithm that takes catheter shape and location data into consideration, the processor (56) can be further configured to (a) identify one (210) of the anatomical regions and classify all of the ablation points (220, 230) as belonging to the identified anatomical region, and (b) present on the anatomical map (200) respective ablation tags (222A) according to the classified ablation points (220, 230) and catheter shape and location data.

It will be appreciated that the examples described above are cited by example and that the present disclosure is not limited to what has been shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art.

For example, features, materials, characteristics, or groups described in conjunction with a particular aspect, arrangement, or example are to be understood to be applicable to any other aspect, arrangement or example described in this section or elsewhere in this specification unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive.

While operations may be depicted in the drawings or described in the specification in a particular order, such operations need not be performed in the particular order shown or in sequential order, or that all operations be performed, to achieve desirable results. Other operations that are not depicted or described can be incorporated in the example methods and processes. For example, one or more additional operations can be performed before, after, simultaneously, or between any of the described operations. Further, the operations may be rearranged or reordered in other implementations. Those skilled in the art will appreciate that in some arrangements, the actual steps taken in the processes illustrated and/or disclosed may differ from those shown in the figures. Depending on the arrangement, certain of the steps described above may be removed, others may be added. Furthermore, the features and attributes of the specific arrangements disclosed above may be combined in different ways to form additional arrangements, all of which fall within the scope of the present disclosure.

The scope of the present disclosure is defined only by reference to the following claims.

## Claims

1. A method for generating an ablation map of a cardiac chamber, comprising:
receiving an anatomical map (200) of wall tissue (202) of at least a portion of the cardiac chamber, the map comprising different anatomical regions (210, 212);
receiving a plurality of ablation points (220, 230) generated by a multi-electrode catheter (14);
using a classification algorithm that considers catheter (14) shape data, identifying an anatomical region (210, 212) and classifying all of the plurality of ablation points (220, 230) as belonging to the identified anatomical region (210) ; and
presenting on the anatomical map (200) respective ablation tags (222A) according to the classified ablation points (220, 230) and the catheter shape data.

2. The method according to claim 1, wherein using the classification algorithm comprises identifying the anatomical region by determining that a majority of the ablation points (220, 230) falls in that region.

3. The method according to claim 1, wherein using the classification algorithm comprises identifying the anatomical region (220) by deeming that a centroid (250) location of the ablation points falls in that region.

4. The method according to claim 1, wherein using the classification algorithm comprises:
assigning a weight to each ablation point based at least in part on the catheter (14) shape data; and
identifying the anatomical region (210) based at least in part on a subset of ablation points (220, 230) yielding the highest total weights.

5. The method according to claim 1, wherein using the classification algorithm comprises identifying the anatomical region (210) by determining a probability of the ablation points (220, 230) belonging to that region (210), the probability calculated based on distances of each of the plurality of ablation points from the anatomical region on the map (200).

6. The method according to claim 1, wherein using the classification algorithm comprises training a machine learning (ML) based algorithm based on a data set of labeled ablation points and using the trained ML-based algorithm to infer the anatomical region of the received plurality of ablation points (220, 230).

7. The method according to claim 6, wherein training the machine learning (ML) model comprises training the model with data labeled using one or more given classification algorithms among the list of: a majority voting classification algorithm, a centroid location classification algorithm, a distance-based probabilistic classification algorithm, and a weighted ablation-points-based classification algorithm.

8. A system (10) for generating an ablation map of a cardiac chamber, comprising:
a memory (57) configured to store a classification algorithm;
and a processor (56), which is configured to:
receive an anatomical map (200) of wall tissue (202) of at least a portion of the cardiac chamber, the map comprising different anatomical regions (210, 212);
receive a plurality of ablation points (220, 230) generated by a multi-electrode catheter (14);
using the classification algorithm that considers catheter shape data, identify an anatomical region and classify all of the plurality of ablation points (220, 230) as belonging to the identified anatomical region; and
present on the anatomical map (200) respective ablation tags (222A) according to the classified ablation points (220, 230) and the catheter shape data.

9. The system (10) according to claim 8, wherein the processor (56) is configured to use the classification algorithm by identifying the anatomical region (210) by determining that a majority of the ablation points (220, 230) falls in that region.

10. The system according to claim 8, wherein the processor (56) is configured to use the classification algorithm by identifying the anatomical region (210) by deeming that a centroid location of the ablation points (220, 230) falls in that region.

11. The system according to claim 8, wherein the processor (56) is configured to use the classification algorithm by:
assigning a weight to each ablation point based at least in part on the catheter shape data; and
identifying the anatomical region (210) based at least in part on a subset of ablation points (220, 230) yielding the highest total weights.

12. The system according to claim 8, wherein the processor (56) is configured to use the classification algorithm by identifying the anatomical region (210) by determining a probability of the ablation points (220, 230) belonging to that region, the probability calculated based on distances of each of the plurality of ablation points (220, 230) from the anatomical region (210) on the map.

13. The system according to claim 8, wherein the processor (56) is configured to use the classification algorithm by training a machine learning (ML) based algorithm based on a data set of labeled ablation points (220, 230) and using the trained ML-based algorithm to infer the anatomical region (210) of the received plurality of ablation points (220, 230).

14. The system according to claim 13, wherein the processor (56) is configured to train the machine learning (ML) model by training the model with data labeled using one or more given classification algorithms among the list of: a majority voting classification algorithm, a centroid location classification algorithm, a distance-based probabilistic classification algorithm, and a weighted ablation-points-based classification algorithm.

15. The method according to claim 1 or the system according to claim 8, wherein the anatomical map (200) is an electroanatomical (EA) map.
